# EUROPEAN PATENT APPLICATION

(11) **EP 3 492 119 A2**
(43) Date of publication of application: **05.06.2019**
(21) Application number: 18209314.6
(22) Date of filing: 29.11.2018
(51) Int. Cl.: A61M 1/12, A61M 1/10, A61B 5/00, A61B 5/0215

(54) **SENSOR SYSTEM FOR ENDOVASCULAR PULSATION BALLOON**

(30) Priority: 01.12.2017 US 201715829186
(71) Applicant: Cook Medical Technologies, LLC, Bloomington, IN 47404 (US); Muffin Incorporated d/b/a Cook Advanced Technologies, West Lafayette, IN 47906 (US)
(72) Inventor: Obermiller, F., West Lafayette, IN Indiana 47906 (US); Havel, William, West Lafayette, IN Indiana 47906 (US)
(74) Representative: Leach, Sean Adam

(57) **Abstract**

A system for monitoring the flow of blood within an endoluminal passage is provided comprising a medical device and a sensor. The medical device is implantable within the endoluminal passage and includes a shaft and an expandable segment coupled to the shaft. The expandable segment is movable between a first state and a second state. Movement of the expandable segment is responsive to a change in the fluid pressure external to the expandable segment. The sensor is positioned on the medical device and is adapted to collect information associated with the state of the expandable segment.

## Description

### Technical Field

This disclosure relates to medical devices and, in particular, to sensors for endovascular assemblies for improving vascular compliance of a vessel.

### Background

The statements in this section merely provide background information related to the present disclosure and may not constitute prior art.

Endovascular devices are commonly used in vascular passages when the vascular passageway becomes stiff and loses compliance. When a vascular passageway loses compliance due to, for example, age, congestive heart failure, or atherosclerosis, the vascular passageway stiffens and loses compliance, causing the heart to exert more force to effect the same volume of blood into the vascular passage. It is desirable to have a sensor system associated with endovascular devices to monitor blood flow within the vascular passageway and provide information to adjust parameters of implanted endovascular devices.

### Summary

Further areas of applicability will become apparent from the description provided herein. It should be understood that the description and specific examples are intended for purposes of illustration only and are not intended to limit the scope of the present disclosure.

In one embodiment, a system for monitoring the flow of blood within an endoluminal passage is provided comprising a medical device and a sensor. The medical device is implantable within the endoluminal passage and includes a shaft and an expandable segment coupled to the shaft. The expandable segment is movable between a first state and a second state. Movement of the expandable segment is responsive to a change in the fluid pressure external to the expandable segment. The sensor is positioned on the medical device and is adapted to collect information associated with the state of the expandable segment.

In another embodiment, a system for monitoring the flow of blood within an endoluminal passage is provided including a medical device and a plurality of sensors. The medical device is implantable within an endoluminal passage and includes a shaft and a plurality of expandable segments coupled to the shaft. The plurality of expandable segments are arranged linearly along the shaft. Each of the expandable segments is movable between a first state and a second state. Movement of the expandable segments is responsive to a change in fluid pressure external to the expandable segment. The plurality of sensors are positioned on the medical device and are configured to collect information associated with the states of the plurality of expandable segments.

In yet another embodiment, a method of determining a characteristic of the flow of blood within an endoluminal passage is provided, including implanting a medical device within an endoluminal passage and transmitting a signal generated by a sensor. The medical device includes a shaft, an expandable segment coupled to the shaft, and a sensor positioned on the medical device. The expandable segment is movable between an expanded state and a compressed state. Movement of the expandable segment is responsive to a change in the flow of blood within the endoluminal passage over the expandable segment. The signal is generated by the sensor responsive to the flow of blood in the endoluminal passage over the medical device.

### Brief Description of the Drawings

The embodiments may be better understood with reference to the following drawings and description. The components in the figures are not necessarily to scale. Moreover, in the figures, like-referenced numerals designate corresponding parts throughout the different views.
FIG. 1 illustrates a cross-sectional view of an aortic passageway including a first example of a medical device;
FIG. 2 illustrates a cross-sectional view of a second example of the medical device;
FIG. 3 illustrates a cross-sectional view of a first example of the expandable segment;
FIG. 4 illustrates a cross-sectional view of a second example of an expandable segment;
FIG. 5 illustrates a cross-sectional view of a third example of an expandable segment;
FIG. 6 illustrates a cross-sectional view of a fourth example of an expandable segment;
FIG. 7 illustrates a cross-sectional view of a fifth example of the expandable segment;
FIG. 8 illustrates a cross-sectional view of a sixth example of the expandable segment;
FIG. 9 illustrates a cross-sectional view of a seventh example of the expandable segment;
FIG. 10 illustrates a cross-sectional view of a eighth example of the expandable segment;
FIG. 11 illustrates a flow diagram of operations to operate a medical device to reduce cardiac back pressure.

The drawings described herein are for illustration purposes only and are not intended to limit the scope of the present disclosure in any way.

### Detailed Description

The following description is merely exemplary in nature and is not intended to limit the present disclosure, application, or uses.

In some examples, a medical device may be placed in the vascular passageway and manually inflated and deflated using a pump which coordinates inflation with the heartbeat of the patient. This configuration can be useful to assist the heart when the patient is stationary, such as when they are asleep or interred at a treatment facility. However, such a device cannot be used without the pumping assembly and therefore restricts the movement of the patient.

In treating particularly serious conditions, such as when a medical device is placed in a non-compliant aorta, frequent or constant monitoring of the blood flow within the endovascular passage may be necessary to ensure the health of the patient.

A system for monitoring the flow of blood within an endoluminal passage is provided comprising a medical device and a sensor. The medical device is implantable within the endoluminal passage and includes a shaft and an expandable segment coupled to the shaft. The expandable segment is movable between a first state and a second state. Movement of the expandable segment is responsive to a change in the fluid pressure external to the expandable segment. The sensor is positioned on the medical device and is adapted to collect information associated with the state of the expandable segment.

One interesting feature of the systems and methods described below may be that the expandable segment and a reservoir portion are configured to provide a passive gradient of flow through the medical device, passively working with the heartbeat of the patient to inflate and deflate the expandable segment with the proper timing, thereby reducing stress on the heart muscles. The lack of an active pump may grant increased freedom of movement to a patient utilizing the medical device.

Another interesting feature of the systems and methods described below may be that the sensor on the medical device may be able to detect and transmit information to the patient and/or physician indicating the status of the blood flow within the endoluminal passage. If the blood flow is non-optimal, the patient may be informed as quickly as possible, allowing the operating parameters of the medical device to be quickly adjusted and avoiding harm to the patient. Furthermore, other aspects of the medical care of the patient may be altered in response to information gathered in the operation of the medical device within the endoluminal passage, such as prescription or alteration of drugs to avoid heart failure.

Yet another interesting feature of the systems and method described below may be that the sensor on the medical device may require no power source located outside the body to gather and transmit data. Such a configuration may grant significant mobility to the patient. In some situations, current may be selectively induced through the sensor remotely from outside the body, ensuring that the patient need not be connected to any wires in order to gather and transmit data.

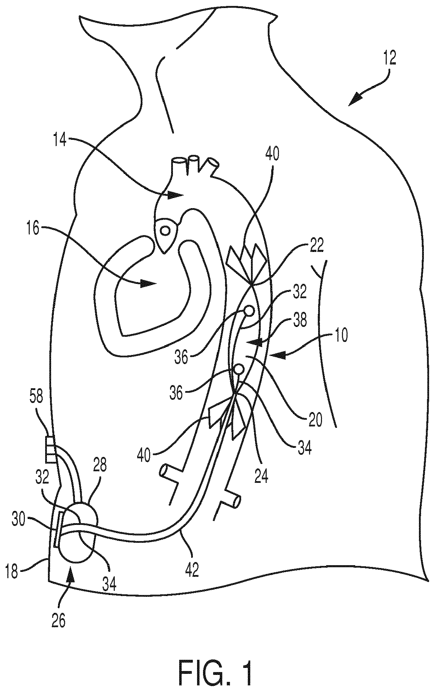

FIG. 1 illustrates a medical device 10 positioned in an endoluminal passage 14 of a patient 12. The endoluminal passage 14 may be any passageway within the body of the patient 12 which is normally compliant to expansion and contraction under healthy conditions. Examples of the endovascular passage 14 may include the aorta, the common iliac arteries, or the subclavian arteries. For example, FIG. 1 illustrates that the medical device 10 may be positioned in the aorta to reduce the effects of vascular non-compliance and reduce the stress on the left ventricle 16 of the heart to pump blood through the body.

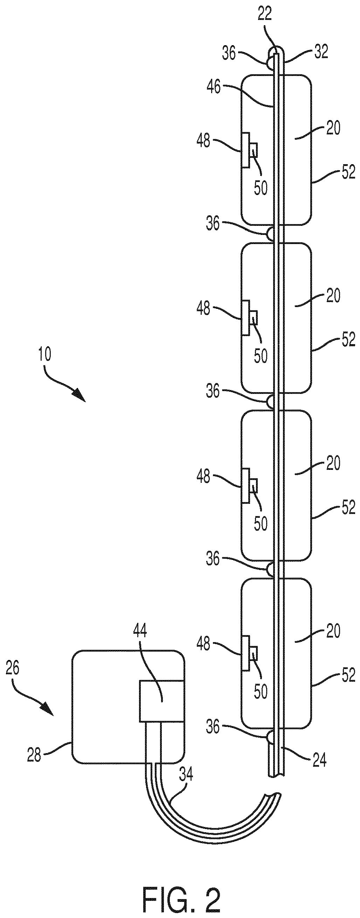

The medical device 10 may include a shaft (46 in FIG. 2), a reservoir portion 26, an expandable segment 20, and electrodes 36. Examples of the shaft 46 may include any object which may be arranged longitudinally within the endoluminal passage 14 and which provides a structure on which to mount the expandable segment 20. Examples of the shaft 46 may include a catheter, a sheath, or a wire guide. The shaft 46 may be tubular or solid.

The expandable segment 20 may be any portion of the medical device 10 which inflates and deflates to control blood flow in the endoluminal passage 14 to increase blood pressure within the endoluminal passage 14. Examples of the expandable segment 20 may include a balloon, a bladder, or a sac. The expandable segment 20 may extend longitudinally along the shaft 46 from a first end 22 to a second end 24. As shown in FIG. 1, the first end 22 of the expandable segment 20 may be positioned closer to the left ventricle 16 of the heart (sometime described as being in a more "proximal", "upstream", or "cranial" position) compared to the second end 24. The second end 24 of the expandable segment 20 may be positioned closer to the iliac arteries (sometimes described as being in a more "distal", "downstream", or "caudal" position) than the first end 22. The expandable segment 20 may be made of any material which can inflate and deflate responsive to blood flow and prevent fluid from inside the expandable segment 20 from leaking into the endoluminal passage 14, such as rubber or a polymer. The expandable segment 20 may be inflated with fluids such as saline or carbon dioxide.

The reservoir portion 26 may be any component capable of receiving and storing fluid outside of the expandable segment 20. Examples of the reservoir portion 26 may include a tube, a sac, an inflatable balloon, or some other container. The reservoir portion 26 may receive fluid from the expandable segment 20 from a tether 42 extending between the expandable segment 20 and the reservoir portion 26. The tether 42 may be any object which couples together and provides fluid communication between the reservoir portion 26 and the expandable segment 20. Examples of the tether 42 may include a tube, a catheter, or a sheath. The tether 42 may be an extension of the shaft 46.

The reservoir portion 26, expandable segment 20, and tether 42 may form a closed fluid system. All of the reservoir portion 26, expandable segment 20, and tether 42 may be filled with a fluid having a density which is less than the density of blood within the endoluminal passage 14. This closed fluid system provides a pumping mechanism when blood flows through the endoluminal passage 14. While the expandable segment 20 is inflated within the endoluminal passage 14, the cross-sectional area that it takes up within the endoluminal passage 14 simulates the changing size of a healthy, compliant endovascular passage 14 and increases the resting pressure of blood within the endoluminal passage 14. The pumping of blood into the endovascular passage 14 by the left ventricle 16 may create a pressure wave which travels downstream from the left ventricle 16. Within this pressure wave, blood flowing through the endoluminal passage 14 may apply increased pressure sequentially from the first end 22 to the second end 24 of the expandable segment 20. Blood flow over a portion of the expandable segment 20 may deflate a portion of the expandable segment 20, causing fluid within the expandable segment 20 to flow into the reservoir portion 26. As the fluid enters the reservoir portion 26, the pressure within the reservoir portion 26 may increase. The increased pressure within the reservoir portion 26 may provide a passive force on the fluid to flow back into the expandable segment 20 as the pressure wave travels past the expandable segment 20 and the pressure of the blood flow diminishes over the expandable segment 20.

The reservoir portion 26 may include a container 28 which may have a fixed volume or which may be inflatable. The container 28 may be made of any material capable of retaining the fluid delivered from the expandable segment 20, such as rubber or a polymer. The reservoir portion 26 may be located within the endoluminal passage 14, inside the body of the patient 12 but outside the endoluminal passage 14, or outside the body of the patient 12 altogether. In some embodiments, the reservoir portion 26 may be located within or directly alongside the expandable segment 20. In such arrangements, a tether 42 may not be needed. In some embodiments, the reservoir portion 26 may include a port 58 coupled to the container 28. The port 58 may extend through the skin 18 of the patient 12. The port 58 may be used to add fluid to the closed fluid system of the expandable segment 20, the reservoir portion 26, and the tether 42, or may be used to remove fluid from the closed fluid system. Adjusting the amount of fluid within the closed fluid system may control the range pressures within the expandable segment 20 and the range of inflation or deflation that the expandable segment 20 experiences.

The medical device 10 may also include a mounting element 40 which fixes the position of the expandable segment 20 within the endoluminal passage 14. The mounting element 40 may be any object which is expandable to grip the walls of the endoluminal passage 14. Examples of the mounting element 440 may include a self-expanding stent portion or barbs. The mounting element 40 may be arranged anywhere along the length of the shaft 46 or expandable segment 20, such as, for example, at the first end 22 and the second end 24 of the expandable segment 20. In some embodiments, the expandable segment 20 may include a mounting element 40 proximate to the first end 22 of the expandable segment 20 to prevent downstream movement of expandable segment 20 as blood flows through the endoluminal passage 14. In other embodiments, the expandable segment 20 may include an additional mounting element 40 proximate the second end 24 of the expandable segment 20 to better fix the position of the expandable segment 20 within the endoluminal passage 14.

The electrodes 36 may be located anywhere along the medical device 10 within the endoluminal passage 14. For example, the electrodes 36 may be located on the surface (52 in FIG. 2) of the expandable segment 20 or may be located on the shaft 46 at the first end 22 and the second end 24 of the expandable segment 20. The electrodes 36 may be any devices which, when an electrical current is applied to them, generates an electromagnetic field between them. Examples of the electrodes 36 may be polarizable electrodes or non-polarizable electrodes. The electrodes 36 may be made of any material suitable to create an electrical potential across the electrodes 36 and which is stable and resistant to corrosion, such as carbon, platinum, or platinum iridium. The material of the surface 52 of the expandable segment 20 and the fluid within the expandable segment 20 may both have higher impedances than the blood within the endoluminal passage 14. Therefore, the lower impedance to current between the electrodes 36 may indicate that the portion of the expandable segment 20 between the two electrodes 36 is deflated or deflating. Similar, an increase in the impedance between the two electrodes 36 may indicate that the portion of the expandable segment 20 between the two electrodes 36 is inflated or inflating.

In order to operate, the electrodes 36 may require an electrical current source. The electrical current source may be any device which is configured to provide a flow of electrons to the electrodes 36. The electrical current source may provide an alternating current or a direct current. In some embodiments, the electric current should not result in heating or stimulation of tissue of the patient 12. For example, an alternating current at a frequency of about 1 kHz may be used to prevent electrical stimulation of tissue. Examples of the electrical current source may be a battery (44 in FIG. 2), or a magnetically activated induction coil 30. As shown in FIG. 1, the induction coil 30 may be located close to the skin 18 of the patient 12. A physician or operator may induce a current into the induction coil 30 across the skin 18 without puncturing the skin 18 to activate the electrodes 36 and determine the impedance in the circuit.

The induction coil 30 may be electrically coupled to the electrodes by a pair of wires 32, 34 which form a circuit with the electrodes 36. The first wire 32 may deliver current the induction coil 30 to the first electrode 36 and the second wire 34 may return current from the second electrode 36. The wires 32, 34 may be made of any material suitable to conduct electricity, such as copper or gold. In some embodiments, the induction coil 30 may be coupled to the reservoir portion 26. In such embodiments, the wires 32, 34, may run through or along the reservoir portion 26 and tether 42 to reach the shaft 46. The wires 32, 34 may run along or through the shaft 46 to reach the electrodes 36.

Other sensors may be used instead of or alongside the electrodes 36 to sense the inflated state of the expandable segment 20.

FIG. 2 illustrates the medical device 10 having a plurality of expandable segments 20 arranged linearly along the shaft 46. Each of the plurality of expandable segments 20 may be moveable between an inflated and deflated state, responsive to a change in the fluid pressure external to the expandable segment 20. The expandable segments 20 are arranged such that as a pressure wave in the blood flow travels through the endoluminal passage 14, the each expandable segments 20 will sequentially deflate and then reinflate as the pressure wave passes. Sequential deflation of the expandable segments 20 may maintain a more even resting blood pressure within the endoluminal passage 14, further decreasing the pumping strain on the left ventricle 16.

As shown in FIG. 2, in some embodiments, electrodes 36 may be arranged longitudinally along the shaft 46 of the medical device 10, positioned on the shaft 46 between each of the expandable segments 20. In such a configuration, impedance between each pair of electrodes 36 may be measured, indicating when each of the expandable segments 20 is deflated and reinflated. Information related to the inflation and deflation of the expandable segments 20 may be useful in ensuring that the medical device 10 is operating effectively. Furthermore, if one of the expandable segments 20 tears or forms a leak, the change in impedance may be readily apparent to an operator.

In some embodiments, the electrical current source for the electrodes 36 may be the battery 44, as shown in FIG. 2. The battery 44 may be positioned anywhere along the medical device 10, such as alongside the shaft 46, expandable segments 20, alongside reservoir portion 26, inside the container 28, or external to the body 12. If the amount of potential electrical current of the battery 44 exceeds the amount of current required to operate the medical device 10 for its entire expected use, the battery 44 may be changed periodically. In such a configuration, the battery 44 may be located close to the skin 18 or to a port 58, to allow easy replacement or charging of the battery 44. The battery 44 may be coupled in parallel or in sequence to the plurality of electrodes 36 through the first wire 32 and the second wire 34.

The medical device 10 may further include one or more piezoelectric sensors 48. The piezoelectric sensor 48 may be any material which may release an electric charge in response to mechanical stress. Examples of the piezoelectric sensor 48 may include a ceramic material, a crystal, or a biological material. The piezoelectric sensor 48 may be positioned on the expandable segment 20 to detect whether the expandable segment 20 is inflating or deflating. For example, the piezoelectric sensor 48 may be bonded to the surface 52 of the expandable segment 20 exposed to the endoluminal passage 14 or within the interior 38 of the expandable segment 20. Alternatively, the piezoelectric sensor 48 may be embedded within the surface 52 of the expandable segment 20. When the expandable segment 20 inflates or deflates, the surface 52 of the expandable segment 20 may expand or contract, causing the piezoelectric sensor 48 to also expand or contract. Compression or expansion of the piezoelectric sensor 48 may release a charge which can be used to power a transmitter 50 electrically coupled to the piezoelectric sensor 48 to transmit a signal. The signal generated by the piezoelectric sensor 48 may be received by a receiver (not shown) to indicate to the operator the status of the expandable segment 20. The transmitter 50 may be physically coupled to the piezoelectric sensor 48 or may be positioned elsewhere in the medical device, such as the shaft 46, and only electrically coupled the piezoelectric sensor 48. The transmitter 50 may be electrically powered only by the piezoelectric sensor 48 or may be powered by a different electrical current source, such as the battery 44.

As shown in FIG. 2, the medical device 10 may contain a plurality of piezoelectric sensors 48 in a variety of configurations. The piezoelectric sensors 48 may be spaced such that each expandable segment 20 contains one piezoelectric sensor 48. Alternatively, one of the expandable segments 20 may contain multiple piezoelectric sensors 48 arranged longitudinally or radially about the expandable segment 20.

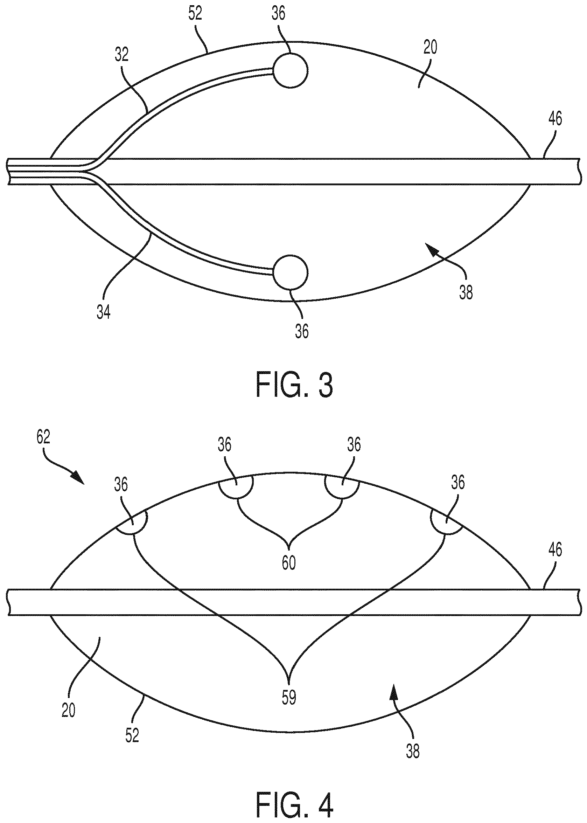

FIG. 3 illustrates an alternative arrangement of the electrodes 36 on the expandable segment 20. On some embodiments, the electrodes 36 may be radially offset on the surface 52 of the expandable segment 20. In such a configuration, the impedance between the electrodes 36 may decrease as the expandable segment 20 deflates, filling the space around the expandable segment 20 with increased electrically conductive blood and bringing the electrodes 36 physically closer together.

FIG. 4 illustrates an alternative arrangement of the electrodes 36 in to an electrode array 62. The electrode array 62 may be any combination of three or more electrodes arranged on the medical device 10. In some embodiments, the electrode array 62 may be a tetrapolar system of electrodes 36, as shown in FIG. 4. The electrode array 62 may include a first pair 59 of electrodes 36 to pass current through the blood within the endoluminal passage 14, and a second pair 60 of electrodes 36 to detect the potential across the blood. The second pair 60 of the electrodes 36 may be partially or entirely arranged in between the first pair 59 of the electrodes 36.

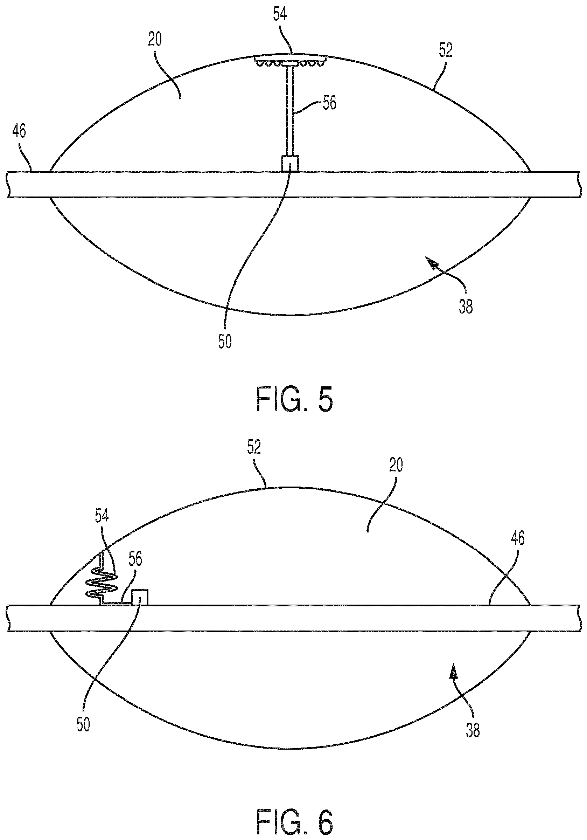

FIG. 5 illustrates the expandable segment 20 having an inductor capacitor (L-C) sensor 54 positioned on the expandable segment 20. The inductor capacitor sensor 54 may be any device which contains at least two parallel coil separated by a dielectric. The inductor capacitor sensor 54 may be positioned on the expandable segment 20 to detect whether the expandable segment 20 is inflating or deflating. For example, the inductor capacitor sensor 54 may be bonded to the surface 52 of the expandable segment 20 exposed to the endoluminal passage 14 or within the interior 38 of the inductor capacitor sensor 54. Alternatively, the inductor capacitor sensor 54 may be embedded within the surface 52 material of the expandable segment 20. The electrical combination within the inductor capacitor sensor 54 may create a resonant circuit that resonates at a particular frequency. As shown in FIG. 3, the coils of the inductor capacitor sensor 54 may be arranged parallel to the surface 52 of the expandable segment 20. When the expandable segment 20 inflates or deflates, the surface 52 of the expandable segment 20 may expand or contract, causing the coils of the inductor capacitor sensor 54 to also expand or contract. Compression or expansion of the coils of the inductor capacitor sensor 54 may increase or decrease the capacitance of the inductor capacitor sensor 54 and may therefore change the resonant frequency of the inductor capacitor sensor 54. An RF signal may be applied to the inductor capacitor sensor 54 from inside or outside the endoluminal passage 14 to cause the inductor capacitor sensor 54 to echo back a detectable signal. From this signal, the capacitance of the inductor capacitor sensor 54 may be calculated, indicating the inflation or deflation of the expandable segment 20.

Alternatively, in some embodiments, the inductor capacitor sensor 54 may be electrically coupled to a transmitter 50 to transmit a signal indicating the capacitance of the inductor capacitor sensor 54. The signal generated by the inductor capacitor sensor 54 may be received by the receiver to indicate to the operator the status of the expandable segment 20. The transmitter 50 may be physically coupled to the inductor capacitor sensor 54 or may be positioned elsewhere in the medical device, such as the shaft 46, and only electrically coupled to the inductor capacitor sensor 54. The transmitter 50 may be electrically powered by a different electrical current source, such as the battery 44.

As shown in FIG. 6, the coils of the inductor capacitor sensor 54 may be arranged such that, a first portion of the inductor capacitor sensor 54 may be coupled to the surface 52 of the expandable segment 20, and a second portion of the inductor capacitor sensor 54 may be coupled to some other component of the medical device 10, such as the shaft 46. In such a configuration, when the expandable segment 20 inflates, the coils of the inductor capacitor sensor 54 may expand, changing the inductance of the inductor capacitor sensor 54. Alternatively, when the expandable segment 20 deflates, the coils of the inductor capacitor sensor 54 may compress, changing the inductance of the inductor capacitor sensor 54.

The electrical current generated within the inductor capacitor sensor 54 may be generated from a variety of sources. For example, a stationary magnet may be arranged within the medical device 10 near the coils of the inductor capacitor sensor 54 to allow expansion or compression of the coils within the magnetic field of the magnet to generate the electrical current. Alternatively, a rotating or otherwise moving magnet could generate a magnetic field which could induce the electrical current within the inductor capacitor sensor 54. The electrical current could also be provided to the inductor capacitor sensor 54 directly through a power source such as the battery 44.

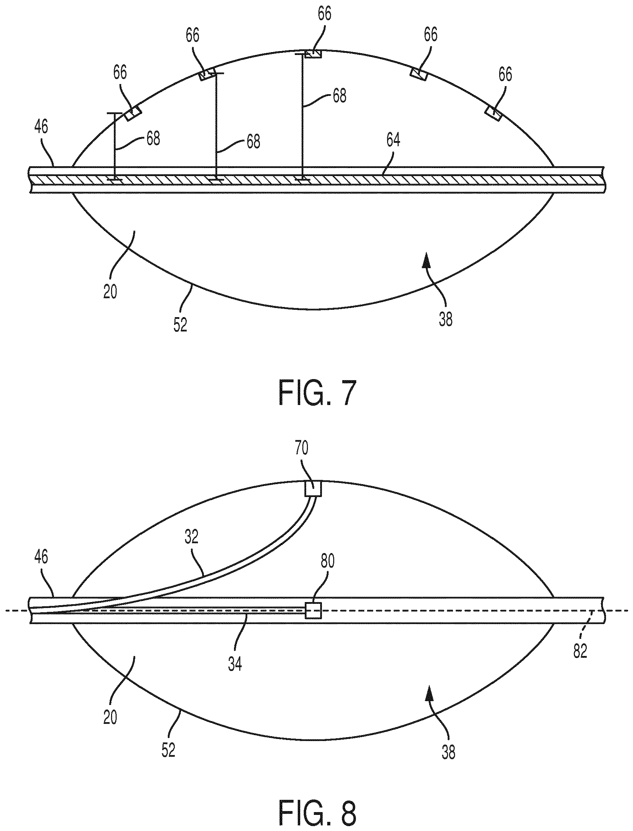

FIG. 7 illustrates the expandable segment having a plurality of markings 66. In some embodiments, the inflation or deflation of the expandable segment 20 may be indicated by the markings 66. The markings 66 may be any feature on the expandable segment 20 which allows non-invasive visualization of the expandable segment 20 from outside the body 12 of the patient. For example, the markings 66 may comprise indentations on the surface 52 of the expandable segment 20 which have a shape, such as a rectangle or triangle, which reflects acoustic energy differently than the surface 52 of the expandable segment 20. Such indentation markings 66 would be visible under ultrasonic visualization to determine the inflated or deflated state of the expandable segment 20. The markings 20 may be placed at a point on the expandable segment 20 which, while inflated, extends furthest from the shaft 46 of the medical device 10. Alternatively or in addition, markings 66 may be spread across the length of the surface 52 of the expandable segment 20.

Another possible embodiment may include metallic markings 66 coupled to the surface 52 of the expandable segment 20. The metallic markings 66 may be located on the outside of the surface 52, within the interior 38 of the expandable segment 20, or embedded within the surface 52 of the expandable segment. The metallic markings 66 may be any metal having a density higher than the other materials of the expandable segment 20. Metallic markings 66 may be used to determine the state of the expandable segment 20 under a variety of visualization techniques including ultrasound and x-ray. Non-ferrous metals may be used in the metal markings 66 to allow visualization under magnetic resonance imaging.

The medical device may also include shaft markings 64 on the surface of or imbedded within the shaft 46 of the medical device 10. The shaft markings 64 may be of the same type as the markings 66 on the expandable segment 20. When visualized, a distance 68 between the shaft markings 64 and the markings 66 on the expandable segment 20 may be used to measure the inflated or deflated state of the expandable segment 20.

FIG. 8 illustrates the expandable segment 20 having a plurality of accelerometers 70, 80. A first accelerometer 70 may be arranged on the surface 52 of the expandable segment 20. The accelerometer 70 may be external to the surface 52 of the expandable segment 20, embedded within the surface 52 of the expandable segment, or arranged on the interior 38 of the expandable segment 20. The first accelerometer 70 may be placed at a point on the expandable segment 20 which, while inflated, extends furthest from the shaft 46 of the medical device 10. The accelerometer 70 may be arranged to measure the acceleration of the surface 52 of the expandable segment which is transverse to an axis 82 defined by the shaft 46 of the medical device 10. The first accelerometer 70 may be able to transmit information about the acceleration of the surface 52 of the expandable segment 20 to determine the state of the expandable segment 20. For example, if the first accelerometer 70 indicates that acceleration outward from the shaft 46 of the expandable segment 20, the operator may determine that the expandable segment 20 is inflating. After accelerating, if the first accelerometer 70 indicates that acceleration has ceased, the operator may determine that the expandable segment 20 is fully inflated.

The medical device 10 may include a second accelerometer 80 on the shaft 46 of the medical device 10. The second accelerometer 80 may be in the interior 38 of the expandable segment 20 on the outer surface of the shaft 46, or may be embedded within the shaft 46. The second accelerometer 80 may be arranged to measure acceleration of the shaft 46 which is transverse to the axis 82 defined by the shaft 46. The second accelerometer 80 may be used in conjunction with the first accelerometer 70 to provide a shaft-stabilized indication of the inflation or deflation of the expandable segment 20. For example, the medical device 10 as a whole, and the shaft 46 in particular, may move transversely within the endoluminal passage 14 due to blood flowing over the medical device 10 or movement of the patient. The acceleration information provided by the second accelerometer 80 coupled to the shaft 46 may be used as a control in determining the inflation or deflation of the expandable segment 20. For example, the transverse acceleration information provided by the second accelerometer 80 may be subtracted from the acceleration information provided by the first accelerometer 70. For improved accuracy, the first accelerometer 70 and the second accelerometer 80 may be transversely aligned with respect to each other.

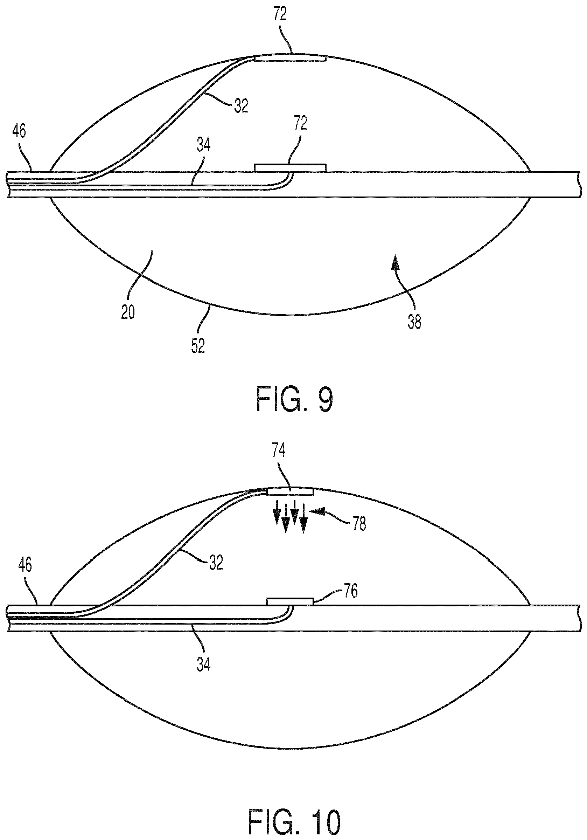

FIG. 9 illustrates the expandable segment 20 including a pair of capacitor elements 72. The capacitor elements 72 may be any electrically conductive material which is capable of storing an electrical charge. Examples of the capacitor elements 72 may include plates or discs and may be made from materials such as copper, gold, or another metal. A first capacitor element 72 may be positioned on the surface 52 of the expandable segment 20, within the interior 38 of the expandable segment 20. A second capacitor element 72 may be positioned on the shaft 42, transversely aligned with the first capacitor element 72 and spaced apart from the first capacitor element 72. The fluid, such as air or carbon dioxide, within the interior 38 of the expandable segment may serve as a dielectric between the capacitor elements 72. The capacitance of the circuit containing the capacitor elements 72 may vary as the expandable segment 20 inflates and deflates, causing the spacing between the capacitor elements 72 to increase and decrease. Accordingly, the varying capacitance of the circuit may be measured and transmitted to determine the inflated or deflated state of the expandable segment 20.

FIG. 10 illustrates the expandable segment 20 including a light emitting source 74 and a light sensor 76. The light emitting source 74 may be any device which emits electromagnetic radiation, such as radio waves, infrared light, visible light, or ultraviolet radiation. Examples of the light emitting source 74 may be a light emitting diode (LED) or a laser. The light sensor 76 may be any device which may receive light emitted by the light emitting source 74. Examples of the light sensor 76 may include an optical sensor or a mirror. In some embodiments the light emitting source 74 may be arranged within the interior 38 of the expandable segment 20 and positioned on one of the surface 52 of the expandable segment 20 or on the shaft 46. The light sensor 76 may also be arranged within the interior 38 of the expandable segment 20 and positioned on the other of the surface 52 of the expandable segment 20 and the shaft 46. The light sensor 76 may be transversely aligned with the light emitting source 74.

In some embodiments, the light emitting source 74 may periodically or continuously emit light 78. The light 78 travels from the light emitting source 74 to the light sensor 76. A circuit containing the light emitting source 74 and the light sensor 76 may be used to measure the time elapsed between when the light 78 was emitted from the light emitting source 74 and when the light 78 was received by the light sensor 76. When the expandable segment 20 is fully inflated, the time necessary for the light 78 to travel from the light emitting source 74 to the light sensor 76 may be greater than the time necessary for the light 78 to travel from the light emitting source 74 to the light sensor 76 when the expandable segment 20 is deflated.

In another embodiment, the light sensor 76 may be a reflective material such as a mirror. In such a configuration, the light sensor 76 may not be connected to an electrical circuit. The light emitting source 74 may also have a sensor to detect when light is received. The light emitting source 74 may periodically or continuously emit light 78. The light 78 may travel through the interior 38 of the expandable segment 20 to reach the reflective light sensor 76. Upon reaching the reflective light sensor 76, the light 78 may reflect against the light sensor 76 and travel back to the light emitting source 74. The light emitting source 74 may receive the light 78 and calculated the travel time between the light emitting source 74 emitting the light 78 and the light emitting source 74 receiving the light 78. This information may be used to determine the degree of inflation of the expandable segment 20 and transmit this information outside the body 12.

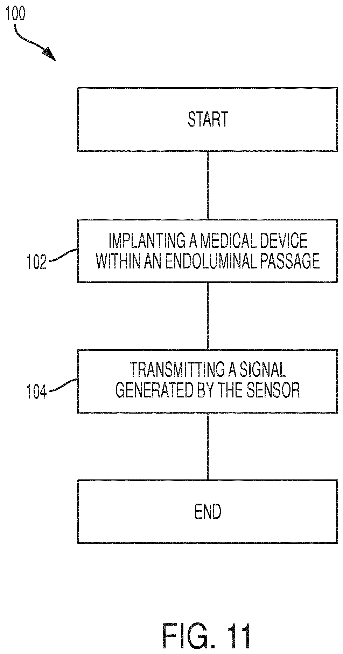

FIG. 11 illustrates a flow diagram of operations to utilize the medical device 10 to reduce cardiac back pressure. The operations (100) may include fewer, additional, or different operations than illustrated in FIG. 6. Alternatively, or in addition, the operations (100) may be performed in a different order than illustrated.

Initially, the method of operations (100) may include implanting the medical device 10 within the endovascular passageway 14 (102). The medical device 10 may include the shaft 46, the expandable segment 20 coupled to the shaft 46, and the sensor 36, 48, 54 coupled to the medical device 10. The expandable segment 20 may be movable between an expanded state and a compressed state, responsive to a change in the blood flow within the endoluminal passage 14 over the expandable segment 20. The method of operations (100) also may include transmitting a signal generated by the sensor 36, 48, 54 (104).

The method (100) may also include electrically charging a circuit including the sensor 36, 48, 54, where the sensor 36, 48, 54 are the electrodes 36 positioned on the surface 52 of the expandable segment 20. The electrodes 36 may longitudinally or radially spaced apart on the surface of the expandable segment 20. The inflated or deflated state of the expandable segment 20 may be determined by measuring the impedance between the electrodes 36.

In some embodiments, the method (100) may also include imaging the endoluminal passage 14 before implanting the medical device 10 to determine a responsiveness of the endoluminal passage 14 to blood flow created by the left ventricle 16. By determining the responsiveness of the endoluminal passage 14, the sensor 36, 48, 54 may be calibrated to the specific endoluminal passage 14 such that when the medical device is implanted, the medical device 10 accurately measures the blood flow within the endoluminal passage and the state of the expandable segment 20.

In some embodiments, the method (100) may also include adjusting the fluid pressure of the medical device 10 based on the signal generated by the sensor 36, 48, 54. If the measurement indicates that the expandable segment 20 is not sufficiently deflating, fluid may be released from the medical device 10 through the port 58 to allow for adequate deflation. Alternatively, where the measurement indicates that the expandable segment 20 is not fully inflating at the resting pressure of the endoluminal passage 14, fluid may be added to the medical device 10 through the port 58 to allow for adequate inflation.

Additionally, the method (100) may also include adjusting a medication dose of a patient based upon information received by the sensor 36, 48, 54. For example, impending heart failure of a patient may be detected by changes in the fluid flowing over the medical device 10 positioned in the endoluminal passage 14.

It will thus be appreciated that there is described herein is a method of determining a characteristic of the flow of blood within an endoluminal passage, comprising: implanting a medical device within an endoluminal passage, the medical device comprising a shaft, an expandable segment coupled to the shaft, and a sensor positioned on the medical device, the expandable segment being movable between an expanded state and a compressed state, wherein movement of the expandable segment is responsive to a change in the flow of blood within the endoluminal passage over the expandable segment; transmitting a signal generated by the sensor responsive to the flow of blood in the endoluminal passage over the medical device (10). This method may further comprise electrically charging a circuit comprising first electrode 36 located at a first position on a surface 52 of the expandable segment 20, and a second electrode 360. Measuring the blood flow over the expandable segment 20 may comprise measuring an impedance between the first electrode 36 and the second electrode 36. The second electrode 36 may be located at a second position on the surface 52 of the expandable segment 20. The first position of the surface 52 of the expandable segment 20 may be longitudinally separated from the second position.

The method may further comprise imaging the endoluminal passage 14 before implanting the medical device 10 to determine a responsiveness of the endoluminal passage 14 to blood flow. Imaging data can thus be obtained, or it can be obtained by retrieval from a memory, or by receipt of data communication. The method may also further comprise calibrating the sensor 36, 48, 54, 70, 72, 76, 80 of the medical device 10 based on the responsiveness of the endoluminal passage 14 (e.g. based on that imaging data) such that the medical device accurately measures the blood flow within the endoluminal passage 14. The method may further comprise adjusting a fluid pressure of the medical device 10 based on the signal generated by the sensor 36, 48, 54, 70, 72, 76, 80. The method may further comprise adjusting a dose of medication to a patient based on the signal generated by the sensor 36, 48, 54, 70, 72, 76, 80.

These and other method of the disclosure may be implemented in any one of the apparatus and systems described and claimed herein. For example the disclosure provides systems configured to perform such methods. One such system comprises a medical device and a sensor. The medical device is implantable within the endoluminal passage and includes a shaft and an expandable segment coupled to the shaft. The expandable segment is movable between a first state and a second state. Movement of the expandable segment is responsive to a change in the fluid pressure external to the expandable segment. The sensor is positioned on the medical device and is adapted to collect information associated with the state of the expandable segment. This can therefore provide a system for monitoring the flow of blood within an endoluminal passage.

To the extent that the methods described herein may be applied to tissues of the human or animal body, it will be appreciated that such methods may not provide any surgical or therapeutic effect. For example, such methods may be applied ex vivo, to tissue samples that are not part of the living human or animal body. In addition, the methods described herein may be practiced on meat, tissue samples, cadavers, and other non-living objects. Their implementation on cadavers may find particular utility in the training of surgeons and medical professionals. Certain methods may also be practiced on training dummies such as a non-living model of the living human or animal body. It will thus be appreciated that the methods described herein may not comprise a method of treatment of the living human or animal body by surgery or therapy.

In addition to the advantages that have been described, it is also possible that there are still other advantages that are not currently recognized but which may become apparent at a later time. While various embodiments have been described, it will be apparent to those of ordinary skill in the art that many more embodiments and implementations are possible. Accordingly, the embodiments described herein are examples, not the only possible embodiments and implementations.

## Claims

1. A system for monitoring the flow of blood within an endoluminal passage, comprising:
a medical device (10) implantable within an endoluminal passage (14), the medical device (10) comprising a shaft (46) and an expandable segment (20) coupled to the shaft (46), the expandable segment (20) being movable between a first state and a second state, wherein movement of the expandable segment (20) is responsive to a change in fluid pressure external to the expandable segment (20); and
a sensor (36, 48, 54, 70, 72, 76, 80) positioned on the medical device (10) configured to collect information associated with the state of the expandable segment (20).

2. The system of claim 1, wherein the sensor (36, 48, 54, 70, 72, 76, 80) comprises a plurality of electrodes (36), including a first electrode (36) positioned at a first end (22) of expandable segment (20) and a second electrode (36) positioned at a second end (24) of the expandable segment (20), wherein the first electrode (36) and the second electrode (36) form an electromagnetic field across a portion of the expandable segment (20).

3. The system of claim 2, further comprising an electrical current source (44) coupled to the first electrode (36) and the second electrode (36), wherein the electrical current source (44) comprises an induction coil (30) implantable within a body (12) of a patient in which a current may be induced from outside the body (12) of the patient.

4. The system of claim 1, 2 or 3, wherein the sensor (36, 48, 54, 70, 72, 76, 80) comprises a first pair (59) of electrodes (36) and a second pair (60) of electrodes (36), wherein the electromagnetic field is formed across the first pair of electrodes (36), and an impedance between the first pair (59) of electrodes (36) is measureable by the second pair (60) of electrodes (36).

5. The system of any preceding claim, wherein the sensor (36, 48, 54, 70, 72, 76, 80) comprises a piezoelectric sensor (48) positioned within the expandable segment (20), the piezoelectric sensor (48) generating an electrical charge responsive to changes in the state of the expandable segment (20).

6. The system of any of claims 1 to 4, wherein the sensor (36, 48, 54, 70, 72, 76, 80) comprises an inductor capacitor sensor (54) positioned within the expandable segment (20), a capacitance of the inductor capacitor sensor (54) being responsive to a change in fluid pressure external to the inductor capacitor sensor (54) to generate an electrical current.

7. The system of any preceding claim, further comprising a transmitter electrically coupled to the sensor (36, 48, 54, 70, 72, 76, 80) and configured to transmit the information associated with the state of the expandable segment (20).

8. The system of any preceding claim comprising a plurality of said expandable segments (20) coupled to the shaft (46), wherein the plurality of expandable segments (20) are arranged linearly along the shaft (46); and
a plurality of said sensors (36, 48, 54, 70, 72, 76, 80) positioned on the medical device (10), the plurality of sensors (36, 48, 54, 70, 72, 76, 80) configured to collect information associated with the states of the plurality of expandable segments (20).

9. The system of claim 8, wherein the plurality of sensors (36, 48, 54, 70, 72, 76, 80) comprises a plurality of electrodes (36), the plurality of electrodes (36) arranged longitudinally along the medical device (10).

10. The system of claim 9, wherein each of the plurality of electrodes (36) is positioned between two of the plurality of expandable segments (20).

11. The system of claim 9, further comprising a reservoir portion (26) coupled to one of the plurality of expandable segments (20), wherein the reservoir portion (26) is sized to only be implantable within a body (12) of a patient outside the endoluminal passage (14); and
an electrical current source (44) electrically coupled to the plurality of electrodes (36), wherein the electrical current source (44) is positioned in the reservoir portion (26).

12. A method of calibrating an implantable medical device for monitoring the flow of blood within an endoluminal passage (14), the method comprising:
obtaining imaging data indicating a responsiveness of the endoluminal passage (14) to blood flow before implantation of the medical device (10);
determining said responsiveness of the endoluminal passage based on the image data;
and, determining an association between:
(a) a change in impedance of electrodes positioned on the surface of an expandable segment of the medical device; and
(b) inflation and/or deflation of an expandable segment (20) of the medical device,
so that the inflated or deflated state of the expandable segment (20) may be determined by measuring the impedance between the electrodes.

13. The method of claim 12 wherein the medical device (10) is configured to be implanted within an endoluminal passage (14), the medical device (10) comprising a shaft (46), the expandable segment (20) coupled to the shaft (46), and a sensor (36, 48, 54, 70, 72, 76, 80) positioned on the medical device (14), the expandable segment (20) being movable between an expanded state and a compressed state, wherein movement of the expandable segment (20) is responsive to a change in the flow of blood within the endoluminal passage (14) over the expandable segment (20);
and the device is configured to transmit a signal generated by the sensor (36, 48, 54, 70, 72, 76, 80) responsive to the flow of blood in the endoluminal passage (14) over the medical device (10), for example wherein the device is provided by the system of any of claims 1 to 11.

14. The method of claim 12 or 13, wherein the electrodes comprise a first electrode (36) located at a first position on a surface (52) of the expandable segment (20), and a second electrode (36), and the change in impedance is measured by electrically charging a circuit comprising said first electrode (36) and said second electrode.

15. The method of claim 14, wherein the second electrode (36) is located at a second position on the surface (52) of the expandable segment (20), the first position of the surface (52) of the expandable segment (20) is separated from the second position, for example separated longitudinally and/or radially from the first position.
